# EUROPÄISCHE PATENTSCHRIFT

(11) **EP 1 404 260 B1**
(45) Veröffentlichungstag und Bekanntmachung des Hinweises auf die Patenterteilung: **15.04.2009**
(21) Anmeldenummer: 02753894.1
(22) Anmeldetag: 04.07.2002
(51) Int. Cl.: A61F 5/058

(54) **SCHIENE FÜR RUHIGSTELLUNGEN IM HANDBEREICH**
SPLINT FOR IMMOBILIZATIONS IN THE AREA OF THE HAND
GOUTTIERE POUR DES IMMOBILISATIONS DANS LA ZONE DE LA MAIN

(30) Priorität: 09.07.2001 AT 54201 U
(43) Veröffentlichungstag der Anmeldung: 07.04.2004
(73) Patentinhaber: Forstner, Wolfgang, 4020 Linz (AT); Foller, Günther, 4031 Linz (AT)
(72) Erfinder: Forstner, Wolfgang, 4020 Linz (AT); Foller, Günther, 4031 Linz (AT)
(74) Vertreter: Hübscher, Helmut
(86) Internationale Anmeldenummer: PCT/AT2002/000194
(87) Internationale Veröffentlichungsnummer: WO 2003/007857

(56) Entgegenhaltungen:
- DE-C- 270 341
- US-A- 1 867 258
- US-A- 4 384 571
- US-A- 4 813 406
- US-A- 5 152 739
- US-A- 6 102 878

## Beschreibung

### Technisches Gebiet

Die Erfindung bezieht sich auf eine Schiene für Ruhigstellungen im Handbereich.

### Stand der Technik

Ruhigstellungen im Handbereich sind u. a. notwendig, wenn Finger oder der Mittelhandbereich Verletzungen aufweisen, beispielsweise Frakturen, Sehnenverletzungen, Luxationen, Distorsionen, Kontusionen bzw. Schnitt-, Quetsch- und Amputationsverletzungen. Ebenso ist eine Ruhigstellung bei Verbrennungen und Erfrierungen, Verätzungen mit Laugen und Säuren und auch bei diversen Erkrankungen der Finger bzw. Hände, z. B. bei Gichtanfällen, Sehnenscheidenentzündungen, Rheumaschüben, Infektionen oder postoperativ nach Dupuytren'schen Kontrakturen, Panaritien und anderen Infektionen im Bereich der Hand notwendig bzw. erwünscht.

Bisher ist in der Praxis lediglich ein brauchbares Schienensystem zur Ruhigstellung des Daumens bekannt, bei dem die übrige Hand, das Handgelenk und die übrigen Finger zumindest eine Teilbeweglichkeit behalten. Bei Verletzungen bzw. bei sonstigen notwendigen Ruhigstellungen im Mittelhandbereich und im Bereich des zweiten bis fünften Fingers werden in der Praxis aus mechanischtechnischen Gründen Schienen verwendet, bei denen das Handgelenk mitfixiert werden muß. Beispielsweise finden hier die sogenannten "Böhlerschienen" Verwendung. Eine Variante zur "Böhlerschiene" mit den gleichen Nachteilen entnimmt man der EP 0 443 388 A1, die über die ganze Länge des Unterarmes reicht, eine zum Unterarm abgewinkelte Handhaltung erzwingt und am Ende der Handtellerstütze Aufnahmeöffnungen zur Schraubbefestigung von gesonderten Fingerstützen aufweist. Eine ähnliche Variante ist Gegenstand der US 4 384 571 A, die einen aus Metall gefertigten und bis weit über das Handgelenk und den unteren Teil des Unterarmes reichenden Schienenkörper besitzt, der durch eine etwa gleiche Länge aufweisende elastische Manschette und zusätzliche Spannbänder befestigt werden kann und eine Schwenkhalterung für eine einzelne Schiene bildet, die mit Hilfe von Stellschrauben in einer Ausrichtungsstellung auf den eben betroffenen Finger fixiert werden kann, wobei bei der Fixierung zwangsweise nur Daumen und Mittelfinger in der natürlichen Strecklage verbleiben, die übrigen Finger aber, insbesondere der kleine Finger bzw. der Zeigefinger, nur in stark ausgespreizter Stellung fixiert werden können. Anlegen, Ausrichten und Fixieren des Schienenkörpers und der Fingerschienen sind umständlich. Die mögliche, mehrmalige Verwendung der Teile ist unhygienisch und die Metallteile behindern zusätzlich notwendige Röntgenaufnahmen.

Dies führt bei allen diesen Schienen in weiterer Folge zu aufwendigen physikotherapeutischen Maßnahmen, um jenen Bereichen der Hand, die nur aus technischen Gründen mitfixiert wurden, insbesondere dem Handgelenk, oder auch unbetroffenen, mitfixierten Fingern die ursprüngliche Beweglichkeit bzw. Leistungsfähigkeit wieder zu verschaffen.

Es wurde zwar in der US 6 102 878 A eine Schiene für Ruhigstellungen im Handbereich mit einem Grundkörper als Auflagestütze für die Handfläche, der mit über den Handrücken führbaren Halterungen an der Mittelhand befestigbar ist, und einen Träger zur wahlweisen Anbringung von Fingerschienen für den zweiten bis fünften Finger bildet, vorgeschlagen, doch hat sich diese Konstruktion nicht als zielführend und in der Praxis einsetzbar erwiesen. Es findet ein rechteckiger Grundkörper in Form einer Metallplatte Verwendung, der mit Hilfe eines einzigen gesonderten Spannbandes, das in der Beuge zwischen Daumen und Zeigefinger die Mittelhand umschließt, befestigt werden soll und der eine Reihe von Aufnahmelöchern für Steckansätze einer Schienenhalterung aufweist, die handseitig je in Zuordnung zu dem zu schienenden Finger angebracht wird und über ein Biegegelenk mit der eigentlichen rinnenförmigen und mit einem Verband zu befestigenden Fingerschiene verbunden ist. Das Gelenk trägt stark auf und behindert die Beweglichkeit der übrigen Finger sowie alle Tätigkeiten mit den weiteren, an sich gesunden Fingern.

Aus der DE 270 341 C ist eine Schiene mit einem ovalen, nur einen Teil der Handfläche abdeckenden Grundkörper bekannt, der doppelwandig unter Freilassung eines Zwischenraumes ausgebildet ist, Luftdurchtrittsöffnungen aufweist und zwischen den beiden Wandungen Ausschiebeführungen für vorher in ihn versenkte Fingerschienen für alle Finger aufweist, die je nach Bedarf herausgezogen werden sollen. Eine zusätzliche Abdeckung reicht bis zu den Ansätzen aller fünf Finger. Die Ausführung ist dickwandig, unstabil, kann nicht einwandfrei befestigt werden und, da von Haus aus alle Schienen angebracht sind, schwer und unhandlich. Auch hier ist eine Ausbildung aus Metall vorgesehen, die Röntgenuntersuchungen unmöglich macht. Die in des US-A-1 867 258 offenbarten Merkmale des Standes der Technik sind im Oberbegriff von Patentanspruch 1 angeführt.

### Darstellung der Erfindung

Aufgabe der Erfindung ist die Schaffung einer Schiene der im einleitenden Teil des die US 6 102 878 A behandelnden Absatzes erwähnten Art, die aber für die Praxis voll brauchbar ist, eine leichte Bauweise bei weitgehender Beweglichkeit nicht betroffener Finger und des Handgelenkes gewährleistet, Röntgenuntersuchungen zuläßt, genau an die jeweils betroffene Hand bzw. deren Finger angepaßt werden kann und allen Anforderungen der Hygiene entspricht.

Die gestellte Aufgabe wird prinzipiell bei einer Schiene der letztgenannten Art dadurch gelöst, daß der in seiner Umrißform und Größe im wesentlichen an die Handfläche angepaßt ist und fingerseitig An- oder Einsteckaufnahmen für Halterungsansätze der Fingerschienen aufweist, wobei die Halterungsansätze und die An- bzw. Einsteckaufnahmen mit ineinandergreifenden, bei Abnahme der Fingerschiene zu Bruch gehenden Verrastungen versehen sind.

Zusätzlich zur Fixierung der Mittelhand am Grundkörper wird durch entsprechende Anbringung von Fingerschienen eine Ruhigstellung nur tatsächlich betroffener Finger bei freier Beweglichkeit des Handgelenkes und weitgehender Beweglichkeit nicht betroffener Finger ermöglicht. Vorzugsweise wird die Schiene in ihren wesentlichen Bereichen, insbesondere im Bereich des Grundkörpers, aus Kunststoffmaterial gefertigt, wobei auf Eigenschaften, wie Röntgenstrahlendurchlässigkeit, Recyclingfähigkeit, Hautverträglichkeit, Beständigkeit gegen Desinfektionsmittel und andere chemische Reizstoffe und auf Farbechtheit besonderer Wert gelegt wird. Selbstverständlich wird berücksichtigt, daß zur Vermeidung von Verletzungen an den berührbaren Teilen der Schiene keine scharfen Kanten, Ecken usw. vorhanden sind und daß Spalten oder ähnliche Nischen bzw. sonstige Sammelräume für Schmutz, Schweiß, Desinfektionsmittel, Salben usw. wegfallen. Durch die besondere Ausbildung der Halterungsansätze und der An- bzw. Einsteckaufnahmen wird eine Wiederverwendung der Schiene - die, wie schon mehrfach erwähnt, unhygienisch und sogar gefährlich wäre - sicher verhindert.

Für nicht von Fingerschienen besetzte An- oder Einsteckaufnahmen des Grundkörpers können Abdeckungen vorgesehen sein, wobei auch hier die Möglichkeit vorhanden sein kann, daß die Einsteckaufnahmen und die Abdeckungen beim Abnehmen zerstört werden.

Derzeit wird wegen der bei geringer Masse erzielbaren ausreichenden Festigkeit eine Ausführung bevorzugt, nach der die Fingerschienen die Grundform von T-Profilen mit flanschseitiger Fingerauflage und sich gegebenenfalls zum freien Ende verjüngendem Steg aufweisen. Es ist auch möglich, von Haus aus längere Fingerschienen vorzusehen und sie auf die jeweils benötigte Länge abzuschneiden. Über geeignete Vorrichtungen können bei entsprechender Materialauswahl auch ursprünglich gerade Fingerschienen in die gewünschte Biegeform gebracht werden.

In der Praxis wird man für verschieden große Hände und vorzugsweise jeweils auch für die rechte bzw. linke Hand zwei oder mehrere Grundkörper zur Auswahl vorsehen, die in der Größe etwa passen. In gleicher Weise sind die Fingerschienen an die Finger- bzw. notwendige Abstützlängen angepaßt bzw. anpaßbar ausgebildet bzw. an den Grundkörper wahlweise gerade oder gebogene Fingerschienen ansetzbar.

Die möglichst einzuhaltende Bedingung, am Handrücken den Knöchel- und Handwurzelbereich von den zur Befestigung des Grundkörpers notwendigen Halterungen freizuhalten, läßt sich dadurch erfüllen, daß als Halterungen für den Grundkörper zwei über Schließen spannbare, von seinen Längsrändern ausgehende Bänder- oder Gurtpaare dienen, von denen das eine innere Band zwischen Daumen und Zeigefinger und das andere zwischen Daumen und Handwurzel herausführbar ist. Es wäre zumindest theoretisch auch möglich, für die Handrückenseite eine schalenförmige Halterung vorzusehen, die mit dem einen Längsrand über ein Scharnier am Grundkörper befestigt ist und am anderen Rand mit dem Grundkörper über eine Spanneinrichtung verbunden werden kann. Einfacher ist aber die erwähnte Ausführung der Halterung aus Bändern oder Gurten, da sich diese leicht an den Handrücken anschmiegen und allenfalls auch das Einlegen von Mull od. dgl. ermöglichen. Als Schließen der Bandpaare können Klettverschlüsse vorgesehen werden.

Herstellungstechnisch wird derzeit eine Ausführung bevorzugt, nach der der Grundfkörper aus einem Trägerteil aus relativ steifem und einer handflächenseitigen Auflage aus weicherem, hautfreundlichem Kunststoffmaterial besteht und die Gurte einteilig aus dem weicheren Kunststoffmaterial angeformt sind.

### Kurze Beschreibung der Zeichnung

Weitere Vorteile des Erfindungsgegenstandes entnimmt man der nachfolgenden Zeichnungsbeschreibung. In der Zeichnung ist der Erfindungsgegenstand beispielsweise dargestellt. Es zeigen
- Fig. 1: eine erfindungsgemäße, für die rechte Hand bestimmte Schiene mit einer Fingerschiene in Ansicht von unten-außen,
- Fig. 2: die Schiene nach Fig. 1 in Seitenansicht,
- Fig. 3: die Schiene nach Fig. 1 in Vorderansicht,
- Fig. 4: einen Schnitt nach der Linie IV-IV der Fig. 1 und
- Fig. 5: in der Fig. 2 entsprechender Darstellungsweise eine Schiene mit gerader Fingerschiene.

### Weg zur Ausführung der Erfindung

Die dargestellten Schienen bestehen jeweils aus einem Grundkörper 1, Gurtenpaaren 2, 3 und je nach Bedarf, wie dargestellt, einer oder mehreren Fingerschienen 4, 4a. Der Grundkörper 1 besteht aus einem inneren Teil 5 aus Hartkunststoff und einer Auflage 6, 7 aus einem weicheren, hautfreundlichen Kunststoff, aus dem auch die anschließenden Gurtpaare 2, 3, die über Klettverschlüsse gespannt und geschlossen werden können, gefertigt sind. Der Grundkörper 1 ist in seiner Außenumrißform im wesentlichen der Form der Handfläche unter Aussparung des Daumenballens angepaßt. Wie ersichtlich ist, kann handseitig eine bombierte Polsterauflage 8 zur besseren Anpassung an die Höhlung der Handinnenfläche vorgesehen werden.

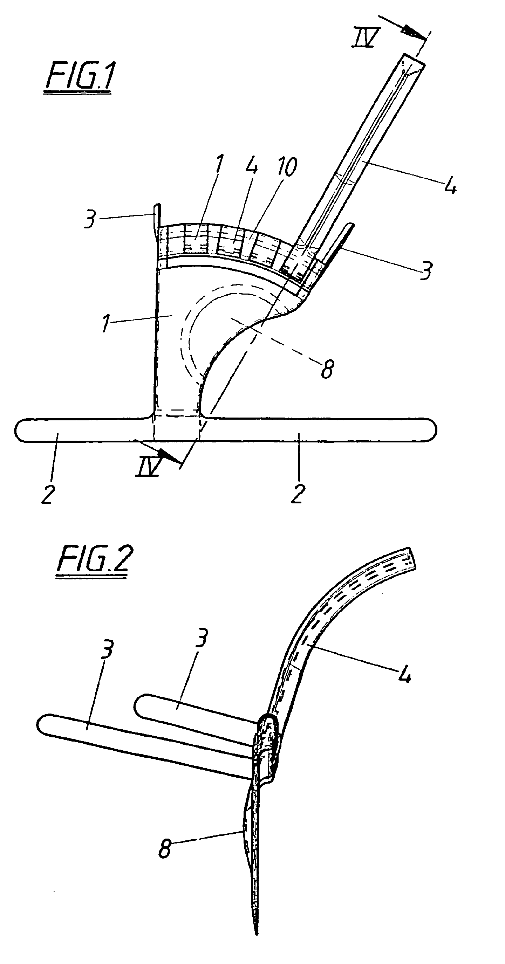

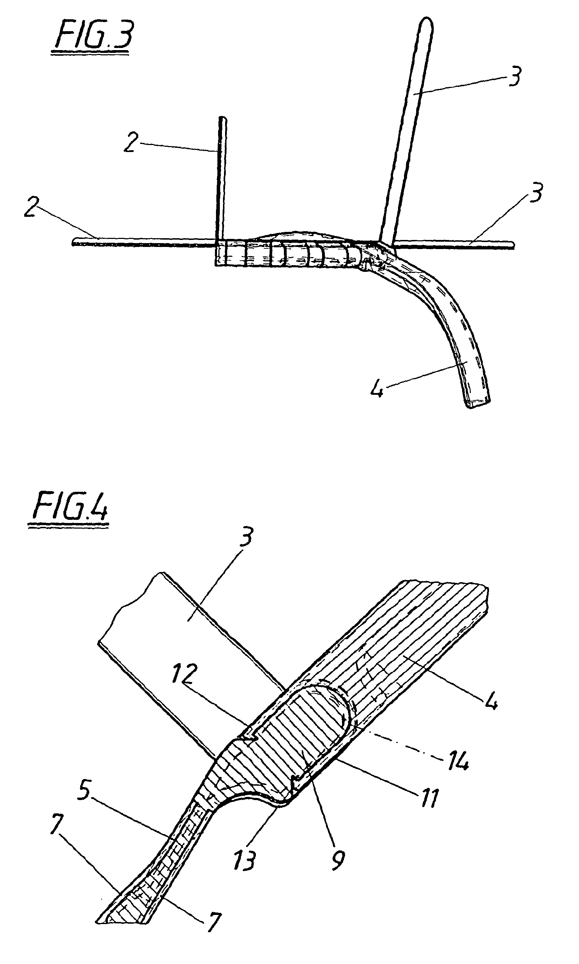

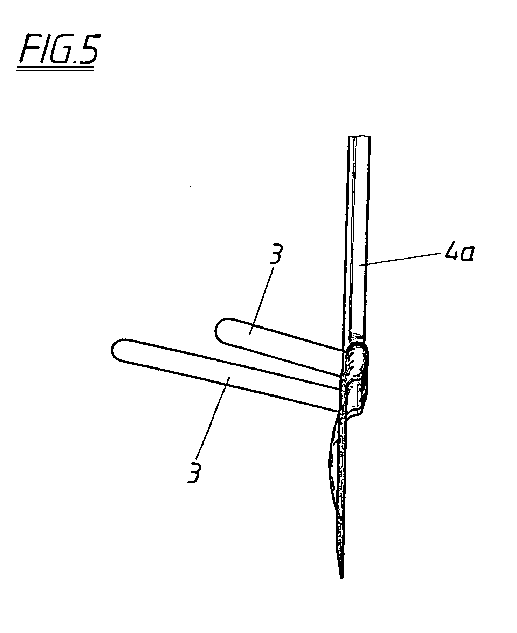

Die Fingerschienen 4, 4a haben die Grundform von T-Profilen mit fingerseitig vorgesehenem Flansch und rückseitigem Steg, der sich zum freien Ende hin verjüngen kann. Nach den Fig. 1 - 3 ist eine gebogene Fingerschiene 4, nach Fig. 5 eine gerade Fingerschiene 4a vorhanden. Am Grundkörper 1 sind fingerseitig Ansteckaufnahmen 9 für je eine Fingerschiene 4 bzw. weitere Fingerschienen für den dritten, vierten und fünften Finger vorhanden. Zwischen diesen Ansteckaufnahmen sind durchgehende Wandungsteile 10 vorgesehen, die angesteckten Fingerschienen 4 die notwendige Seitenstabilität geben. Die Fingerschienen 4, 4a selbst sind mit Halterungsansätzen 11 versehen, die mit widerhakenartigen Rastvorsprüngen 12 in Gegenrasten 13 der Ansteckaufnahmen 9 eingreifen. Für nicht besetzte Ansteckaufnahmen 9 sind gleiche Rastvorsprünge 12 wie die Fingerschienen 4 selbst aufweisende Abdeckungen 14 vorhanden, die außenseitig mit den Oberflächen der Wandungsteile 10 abschließen und gewölbt sind, so daß nicht befestigte Finger auf einer gewölbten, glatten Oberfläche aufruhen.

## Patentansprüche

1. Schiene für Ruhigstellungen im Handbereich bestehend aus Fingerschienen und einem Grundkörper (1) als Auflagestütze für die Handfläche, wobei der Grundkörpermit über den Handrücken führbaren Halterungen (2, 3) an der Mittelhand befestigbar ist und einen Träger zur wahlweisen Anbringung der Fingerschienen (4, 4a) für den zweiten bis fünften Finger bildet, wobei der Grundkörper (1) in seiner Umrißform und Größe im wesentlichen an die Handfläche angepaßt ist und fingerseitig An- oder Einsteckaufnahmen (9) für Halterungsansätze (11) der Fingerschienen (4, 4a) aufweist, **dadurch gekennzeichnet, daß** die Halterungsansätze (11) und die An- bzw. Einsteckaufnahmen (9) mit ineinandergreifenden, bei Abnahme der Fingerschiene zu Bruch gehenden Verrastungen (12, 13) versehen sind.

2. Schiene nach Anspruch 1, **dadurch gekennzeichnet, daß** für nicht von Fingerschienen (4) besetzte An- oder Einsteckaufnahmen (9) des Grundkörpers (1) Abdeckungen (14) vorgesehen sind.

3. Schiene nach den Ansprüchen 1 und 2, **dadurch gekennzeichnet, daß** die Fingerschienen (4, 4a) die Grundform von T-Profilen mit flanschseitiger Fingerauflage und sich gegebenenfalls zum freien Ende verjüngendem Steg aufweisen.

4. Schiene nach einem oder mehreren der Ansprüche 1 bis 3, **dadurch gekennzeichnet, daß** der Grundkörper (1) in Umrißform und Größe an die rechte bzw. linke Handfläche angepaßt ist.

5. Schiene nach einem der Ansprüche 1 bis 4, **dadurch gekennzeichnet, daß** als Halterungen für den Grundfkörper (1) zwei über Schließen spannbare, von seinen Längsrändern ausgehende Bänder- oder Gurtpaare (2, 3) dienen, von denen das eine innere Band zwischen Daumen und Zeigefinger und das andere zwischen Daumen und Handwurzel herausführbar ist.

6. Schiene nach einem der Ansprüche 1 bis 5, **dadurch gekennzeichnet, daß** der Grundkörper (1) aus einem Trägerteil (5) aus relativ steifem und einer handflächenseitigen Auflage (7) aus weicherem, hautfreundlichem Kunststoffmaterial besteht und die Gurte (2, 3) einteilig aus dem weicheren Kunststoffmaterial angeformt sind.

## Claims

1. Splint for immobilisation in the area of the hand, comprising finger splints and a basic body (1) as a support for the palm of the hand, wherein the basic body (1) is adapted to be fixed to the middle of the hand by means of holding elements (2, 3) which can be guided via the back of the hand and forms a carrier for the optional arrangement of the finger splints (4, 4a) for the second to fifth fingers, wherein the basic body (1) is adapted in its contour and size substantially to the palm of the hand and comprises on the finger side placing or insertion receiving areas (9) for holding attachments (11) of the finger splints (4, 4a), **characterised in that** the holding attachments (11) and the placing or insertion receiving areas (9) are provided with fastenings (12, 13) which engage in each other and break upon removal of the finger splint.

2. Splint according to claim 1, **characterised in that** covers (14) are provided for placing or insertion receiving areas (9) of the basic body (1) which are not occupied by finger splints (4).

3. Splint according to claims 1 and 2, **characterised in that** the finger splints (4, 4a) have the basic shape of T profiles with flange-side finger support and a web which is tapered as appropriate towards the free end.

4. Splint according to one or more of the claims 1 to 3**, characterised in that** the basic body (1) is adapted in its contour and size to the right or left palm.

5. Splint according to one of the claims 1 to 4, **characterised in that** two pairs of bands or straps (2, 3) serve as holding elements for the basic body (2) which can be tensioned by closing and come from the longitudinal edges of the basic body (1), one of said pairs of bands or straps (2, 3) being an inner band between thumb and index finger and the other between thumb and wrist.

6. Splint according to one of the claims 1 to 5, **characterised in that** the basic body (1) consists of a carrier part (5) made of relatively stiff plastic material and a support (7) on the side of the palm of the hand made of a softer, skin-friendly plastic material and the straps (2, 3) are formed integrally from the soft plastic material.

## Revendications

1. Gouttière pour des immobilisations dans la zone de la main, composée de gouttières pour les doigts et d'un corps de base (1) faisant office d'appui pour la surface de la main, où le corps de base est susceptible d'être fixé, à l'aide de fixations (2, 3), pouvant être guidées sur le revers de la main, sur le métacarpe et forme un support pour le montage, au choix, des glissières pour les doigts (4, 4a) pour le deuxième jusqu'au cinquième doigt, où le corps de base (1), dans sa forme de profil et sua taille, est sensiblement adapté à la paume de la main et présente des logements d'attachement ou d'enfichage (9), côté doigt, pour des appendices de fixation (11) des gouttières pour les doigts (4, 4a), **caractérisée en ce que** les appendices de fixation (11) et les logements d'attachement ou d'enfichage (9) sont munis d'encliquetages (12, 13) s'engageant les uns dans les autres, finissant par se rompre lors de l'enlèvement des gouttières pour les doigts.

2. Gouttière selon la revendication 1, **caractérisée en ce que** des recouvrements (14) sont prévus pour les logements d'attachement ou d'enfichage (9) du corps de base (1) non occupés par des gouttières pour les doigts (4).

3. Gouttière selon les revendications 1 et 2, **caractérisée en ce que** les gouttières pour les doigts (4, 4a) présentent la forme de base de profilé en T, avec un reposoir à doigts côté bride, et une nervure allant en s'effilant, le cas échéant jusqu'à l'extrémité libre.

4. Gouttière selon l'une ou plusieurs des revendications 1 à 3, **caractérisée en ce que** la forme de profil et la taille du corps de base (1) sont adaptées à la paume de la main droite ou gauche.

5. Gouttière selon l'une ou plusieurs des revendications 1 à 4, **caractérisée en ce que** l'on utilise comme fixations pour le corps de base (1) deux paires de bandes ou ceintures (2, 3), pouvant être tendues par fermeture, partant de ses bords longitudinaux, dont une bande intérieure est susceptible d'être sortie entre le pouce et l'index et l'autre est susceptible être sorti entre le pouce et le carpe.

6. Gouttière selon l'une ou plusieurs des revendications 1 à 5, **caractérisée en ce que** le corps de base (1) est forme d'une partie support (5), en un matériau synthétique relativement rigide, et d'un reposoir (7), situé du côté du plat de la main, réalisé en un matériau synthétique plus souple et acceptable pour la peau, et les bandes ou ceintures (2, 3) sont formées d'une seule pièce, à partir du matériau synthétique plus souple.
